# EUROPEAN PATENT SPECIFICATION

(11) **EP 2 432 450 B1**
(45) Date of publication and mention of the grant of the patent: **31.07.2019**
(21) Application number: 10720694.8
(22) Date of filing: 13.05.2010
(51) Int. Cl.: A61K 9/00, A61K 9/70, A61Q 19/00, A61Q 17/00, A61K 8/02

(54) **SINGLE-USE, DISPOSABLE STRIP FOR APPLICATION OF TOPICAL COMPOSITIONS**
EINWEGSTREIFEN ZUR EINMALIGEN ANWENDUNG VON TOPISCHEN ZUBEREITUNGEN
BANDE JETABLE À USAGE UNIQUE POUR APPLICATION DE COMPOSITIONS TOPIQUES

(30) Priority: 22.05.2009 US 180463 P
(43) Date of publication of application: 28.03.2012
(73) Proprietor: Wyeth LLC, New York, NY 10017-5755 (US)
(72) Inventor: GUO, Jian-Hwa, Richmond Virginia 23233 (US)
(74) Representative: Pfizer
(86) International application number: PCT/US2010/034656
(87) International publication number: WO 2010/135131

(56) References cited:
- WO-A1-00/64409
- WO-A1-01/41746
- WO-A1-98/17251
- WO-A2-2008/055107
- WO-A2-2008/133982
- JP-B2- 4 157 878
- US-A- 3 308 837
- US-A1- 2004 131 662

## Description

### FIELD

Single use disposable topical lip treatment strips are provided. The treatment strips include strips for applying lip treatment compositions.

### BACKGROUND

Topical compositions such as lip balms, for example, are typically formed into sticks and applied directly from the stick, or dispensed from jars or tubes. In stick form the composition is applied directly to the lips from the tube with end of the stick being exposed to the environment and touching the lips each time composition is applied from the tube which provides the opportunity for contamination from a previous use. Jar and tube dispensers provide even more opportunity for contamination as the fingers are usually used to apply the composition.

The cosmetic industry has used devices know a "lipstick testers" for potential customers to sample product. These are typically solid supports of an impermeable paper or cardboard with a small amount of lipstick deposited on the surface. (See, for example, US Patent 5,396,913.) The composition being transferred (e.g. the lipstick) is a single homogenous substance. As the main purpose of these devices is to give the potential customer an opportunity to try the color of the cosmetic against their skin, it is only necessary to transfer a very small amount of the composition. Very small amounts of composition are also desirable to prevent messiness in storage and distribution.

Lipstick testers have material deposited on one surface of the solid support and typically have a film covering the composition to prevent undesired transfer of the composition in storage and distribution. The solid support may have impermeable projections to facilitate positioning and removing the film for use. (See, for example, U.S. Patents 5,396,913 and 4,995,408.) One-piece prepared sheets for applying cosmetic make-up to lips are also disclosed in U.S. Patent 3,308,837.

Since lipstick testers are coated with composition on only one surface, they must be bent or folded in some manner or turned over to apply composition to both lips.

Support bodies onto which a base material is deposited on each surface allowing easy application of a lip treatment are disclosed in JP 4 157878. However, further single use devices that could apply types of compositions and amounts thereof suitable to deliver topical lip treatments are desirable. Furthermore, generally a single use device that could be used to apply other types of topical treatments with minimum mess and minimization of the opportunity for contamination is also desirable.

### SUMMARY

The invention provides a topical lip treatment strip comprising a substrate having two surfaces with a topical lip treatment composition carried thereupon. The topical composition is deposited onto the two surfaces of the substrate and thus available for simultaneously treating both lips of a subject. In particular the invention is directed to a topical lip treatment strip comprising:a substrate comprising two surfaces; and a topical lip treatment composition wherein the topical lip treatment composition is deposited onto the two surfaces of the substrate; and wherein each strip has a length of 1 cm to 10 cm and a width of 1 cm to 5 cm; wherein one or more grasping portions are provided at the side(s) of the treatment strip on which no topical composition is deposited.

Optionally, the topical composition is at least partially absorbed on the substrate.

The substrate may be selected from natural polymers, synthetic polymers, semi-synthetic polymers, fabrics, paper, woven material, non-woven material and combinations thereof. Optionally, in some embodiments the substrate is biodegradable and/or hydrolyzes in the presence of moisture.

The topical treatment strip is designed for use as lip treatment strips.

The topical composition may comprise at least one ingredient selected from the group consisting of beneficial agents, humectants, moisturizers, sunscreens and active or therapeutic agents and combinations thereof. Optionally, the topical treatment strip may further comprise at least one second topical composition wherein the second topical composition is deposited in a layer over the first topical composition on at least one of the two surfaces of the treatment strip.

The invention provides a method method of making a topical lip treatment strip as defined in claim 1, comprising:preparing a topical lip treatment composition; and depositing the topical lip treatment composition onto the two surfaces of a substrate comprising two surfaces, and avoiding deposition of the composition on the one or more grasping portions. Optionally, a one or more additional layers of the same or different topical compositions may be applied over the first topical composition.

The invention also provides a kit for lip treatment comprising a resealable container having a plurality of topical lip treatment strips deposited therein.

A method of using topical lip treatment strip for treatment of lips is also provided. The method comprises grasping the strip by the grasping portion, positioning the strips between the lips of a user, and pursing the lips against the strip.

### BRIEF DESCRIPTION OF DRAWINGS

The foregoing and other features of the present invention will be more readily apparent from the following description and drawings of illustrative embodiments of the invention wherein like reference numbers refer to similar elements throughout the several views and in which:
FIG. 1 shows a schematic diagram of an exemplary embodiment of manufacture of the topical treatment strips of the invention;
FIG. 2 shows a schematic diagram of an exemplary embodiment of manufacture of the topical treatment strips of the invention having a plurality of topical compositions layered onto the substrate;
FIG. 3 shows a schematic diagram of an exemplary embodiment of manufacture of the topical treatment strips of the invention having topical composition on a single side of the substrate;
FIG. 4 shows a schematic diagram of an exemplary embodiment of manufacture of the topical treatment strips of the invention utilizing a spray system to deposit the topical composition on the substrate;
FIG. 5 shows a schematic diagrams of an exemplary embodiment of manufacture of the topical treatment strips of the invention in which a plurality of strips are produced simultaneously, FIG. 5A is a cross-sectional view; FIG 5B is a front view;
FIG. 6 is a diagram of an exemplary embodiment in which a plurality of strips are formed simultaneously;
FIGs. 7A and 7B show diagrams of two exemplary embodiments of the strips of the invention.

### DETAILED DESCRIPTION OF THE INVENTION

The present invention provides a single use film or strip dosage form for application of a topical composition. The single use dosage form is particularly applicable for topical lip treatment compositions including lip balms, lip balms with sunscreens, and/or lip balms with beneficial and/or therapeutic agents. The single use strip offers advantages over stick lip products by minimizing the contamination associated with re-application over time from a previously used stick. The single use strip offers advantages over application from pots or tubes as it avoids both the contamination and mess associated with using the fingers to smear product on the lips. In an exemplary embodiment, a topical composition is coated onto a flat substrate. The treatment may coat both surfaces of the flat substrate. The coating may cover the entire surface of the substrate or alternatively areas of the substrate may be left uncoated. Uncoated regions of the substrate may serve as grasping portions so that the treatment strip may be grasped with the fingers for removal from a package and/or positioning the strip to apply the treatment with at least minimal, and preferably, no topical composition touching the fingers.

The treatment strip is preferably sized to be a convenient size for the area to be treated. For example, in one embodiment the a treatment strip for lips would have topical composition covering an area on the substrate approximating the length and width of human lips and sufficient uncoated area on the surface of the substrate to allow for grasping the treatment strip with the fingers. In one embodiment for use on the lips, the strip has a length of about 1cm to about 10 cm, alternatively about 2 cm to about 8 cm and alternatively about 2 to about 6 cm; and a width of about 1 to about 5 cm, alternatively about 1 to about 4 cm and alternatively about 1 to about 3 cm. About 50% to about 95% and alternatively about 50% to about 80% of the surface area of at least one side of the strip and preferably of both sides of the strip is coated with treatment composition. The remainder of the surface, which is uncoated, forms the grasping portions. Preferably the two sides of a given strip are coated over approximately the same portion and position on each side of the strip.

In some embodiments, it is desirable that the treatment material penetrate into the substrate to facilitate loading of a sufficient amount of topical composition onto the substrate for one treatment and/or to provide convenient transfer of treatment material to the area to be treated and/or minimize messiness of soft topical compositions, for example.

In some embodiments multiple layers of treatment materials may be applied to both surfaces of the substrate. Some exemplary circumstance in which multiple layers may be desirable include, but are not limited to, the following: Multiple layers may be desirable when the desired treatment includes multiple components two or more of which have some degree of incompatibility. The incompatible ingredients may be placed in separate layers. For treatments in which both hydrophobic and hydrophilic components are desired for use, hydrophilic and hydrophobic components may be put in separate layers. Multiple layers may be desirable if a desired active is sensitive or instable in the presence of water and/or air. The sensitive material may be put in a layer and coated with a barrier layer. While the number of layers on a surface of the substrate is typically one, two or three, in principle there is no restriction to the number of layers that may be applied. Further, if both surfaces of the substrate are coated, there is no requirement that the same number of layers be placed on each surface and/or that the materials forming the layers be the same for both surfaces.

The topical lip composition deposited on the single-use film or strip dosage form may comprise, for example, pharmaceutical actives and/or personal care ingredients and/or cosmetic ingredients and/or beneficial agent. Exemplary specific types of compounds include, but are not limited to, cold sore therapy actives, vitamins, anti-aging ingredients, UV-sun blockers, humectants, moisturizers, wound care compositions, antimicrobial substances, antiviral substances and the like. Other ingredients and/or additives of the type typically included in topical composition may be included in the topical lip composition. Exemplary specific types of compounds include, but are not limited to, waxes, viscosity thickening agents, oils, preservatives, buffering agents, flavors, colorants and the like. The lip composition may comprise multiple components. In one embodiment the topical lip composition may be prepared by blending the components, or some other suitable mixing method prior to coating them onto the substrate. If more than one topical lip composition is to be applied to the substrate, each composition may be prepared separately and then individually layered onto the substrate. Preferably, the topical lip composition is formulated both to give the desired treatment or benefit and to be in a form that transfers readily from the substrate to the area to be treated.

The film or strip substrate may be made of any natural, synthetic and/or semi-synthetic polymers, fabrics, paper, woven or non-woven substrates that can be compressed into a film or strip, for example. In some embodiments it is desirable that the substrate be at capable of absorbing/adsorbing at least a portion of the coating. In one embodiment the substrate preferably does not react with the topical lip composition. Further it is desirable that the substrate maintains structural integrity in the presence of the topical lip treatment and provides sufficient physical support to allow the transfer of the topical composition to the area to be treated.

The topical lip treatment strips may be formed individually. Alternatively, the topical lip composition may be deposited onto a sheet or ribbon substrate which is then cut into individual treatment strips after the topical lip composition is deposited onto the substrate. The size and shape of the individual treatment strip can be customized to provide a strip with proportions optimized for transfer to the site of use of the topical lip composition.

Once formed, the topical lip treatment strips may by packaged to prevent contamination and/or transfer of the topical lip composition in storage or distribution. For instance, each strip may be individually packaged. Alternatively, a plurality of strips may be packaged together in a dispenser. An exemplary dispenser is a dispenser in which the individual strips are easy to remove without disturbing other strips. Additionally, it is desirable that the dispenser have a resealable closure to prevent environmental contamination between removal of strips. Optionally, a coated ribbon of substrate comprising a plurality of treatment strips may be packaged in a closable container equipped with a cutting device, such that the ribbon may be cut into treatment strips at the time of use.

To use the topical lip treatment strip, the strip may be grasped with the fingers and applied to the area to be treated. In embodiments having a grasping portion (e.g. areas on the surface of the strip having no topical composition), the strip is preferably grasped on the grasping portion. In an exemplary embodiment of a lip treatment strip, the strip is used by grasping the strip by the grasping portion, positioning the strip between the lips of a user, and pursing the lips against the strip.

As used herein, "single use form" should be taken to include treatment strips, treatment films and unless explicitly indicated to be otherwise, the terms "film" and "strip" should be considered to be interchangeable and refer to a flat object having both a length and width at least 5 times greater than the thickness of the object. The surface of the strip refers to one of the two surfaces defined by the length and the width of the strip. Preferably treatment strips contain sufficient treatment material for single one-time and/or one dose treatment.

As used herein, "topical treatment" is a semisolid or liquid composition for application to the surface of a mammalian body that may provide protective and/or moisturizing properties, and/or a beneficial agent and or a sunscreen and/or pharmaceutical active to the region of the body to which it is applied. As used herein, unless explicitly indicated to be otherwise, a "topical treatment" and "topical composition" should be considered to be interchangeable.

"Beneficial agents" are ingredients know in the cosmetic industry to provide benefits to the lips or other tissues, but are not recognized by the Food and Drug Administration to be pharmaceutical actives.

The term "active", "active agent", "pharmaceutical active", "active pharmaceutical agent", or "drug" as used herein should be considered to have the same meaning and refer to a substance that is known to have a pharmaceutical effect when administered to a human.

The terms "effective amount" or "therapeutically effective amount" of an active agent or beneficial agent as provided herein are defined as an amount of the agent at least sufficient to provide the desired therapeutic or treatment effect.

A "lip treatment" and "lip balm" as used herein should be considered to have the same meaning and refer to a semisolid composition for application to the lips that may provide protective and/or moisturizing properties, and/or a beneficial agent and/or a sunscreen and/or pharmaceutical active to the lip.

The terms "about" or "approximately" mean within an acceptable range for the particular parameter specified as determined by one of ordinary skill in the art, which will depend, in part, on how the value is measured or determined, e.g. the limitations of the measurement system. For example, "about" can mean a range of up to 10% of a given value.

Percent" or "%" as used herein refers to the percentage by weight of the total composition, unless otherwise specified.

The term "w/w", unless otherwise indicated, means weight of a given component or specified combination of components to total weight of the composition expressed as a percentage.

A designation that a substance is a semisolid should be taken to mean the physical state of the substance in the temperature range of about 20° C to about 40° C.

As used herein, a "butter" or "botanical butter" is a fat and/or oil extract of a plant fruit and/or seed characterized by having emollient properties and a melting point near human body temperature. A butter includes both pure extracts from a plant fruit or seed and/or extract from a plant fruit or seed combined with additional lipid material to achieve the melting point characteristic and/or lubricity. Preferably the lipid material is derived from a botanical source. Exemplary butters include, but are not limited to, mango seed butter, raspberry butter, avocado butter, shea butter, olive butter, kuku butter, monoi butter, peach butter, pistachio butter, coconut butter, cocoa butter, pomegranate butter, rose hip butter, sunflower butter, wheat germ butter, apricot butter, babassu butter, cupuacu butter, kokum butter, hazelnut butter, jojoba butter, sesame butter, soy butter, almond butter, meadowfoam seed butter, black current seed butter and cranberry butter.

A "sensate" as used herein is a composition that initiates a sensory perception such as heating or cooling, for example, when contacted with the skin and/or lips. Sensates typically have high potency and accordingly may yield significant impact at low levels. A sensate may be derived from a natural source or prepared synthetically.

A "vasoconstrictor" means a substance or agent that promotes the constriction of blood vessels.

An "anesthetic" means a substance that is capable of producing a complete or partial loss of feeling.

The term "petrolatum" refers to petroleum jelly, which is a mixture of the softer members of the paraffin or methane series of hydrocarbons, obtained from petroleum as an intermediate product in the distillation. Petrolatum is typically perceived as soothing when applied to the human skin.

The treatment strips are for lip care. For lip treatment strips, at least one lip treatment composition is deposited on a substrate. An exemplary lip treatment composition comprises a wax or other pharmaceutically acceptable vehicle and, optionally, one or more medicaments and/or other active agents and/or one or more beneficial agents. Suitable waxes and pharmaceutical vehicles include, but are not limited to, petrolatum wax; carnauba wax; paraffin wax; white wax; candelilla wax; beeswax; oils, such as arachidyl propionate, cetyl alcohol, isopropyl lanolate, isopropyl myristate, lanolin, mineral oil, light mineral oil, octyldodecanol, oleyl alcohol, ethyl macadamiate, castor oil, jojoba esters, hydrogenated castor oil, hydrogenated vegetable oil, cetyl ricinoleate, propylene glycol, isopropyl palmitate, stearyl alcohol, and volatile and non-volatile silicone oils; and any combination of any of the foregoing. Suitable silicone oils include, but are not limited to, polyphenylmethyl siloxane, dimethicone, cyclomethicone, and any combination of any of the foregoing.

Suitable active agents and/or beneficial agents for lip treatment compositions include, but are not limited to, sunscreens, such as octyl methoxycinnamate, octyl dimethyl p-aminobenzoate, actinoquinol, p-aminobenzoic acid, butyl methoxydibenzoylmethane, beta-carotene, 4-dimethylamino benzoic acid, dioxybenzone, drometrizole, lawsone, sulisobenzene, titanium dioxide, and zinc oxide; skin conditioning agents, including but not limited to, the aforementioned waxes and oils, botanical oils and butters, petrolatum, dimethicone, polymethylsiloxane; emollients and moisturizers. Exemplary moisturizering oils include, but are not limited to, sunflower oil, coconut oil, castor oil, vegetable oil, corn oil, aloe vera oil, canola oil, soybean oil, jojoba oil, olive oil, babassu oil, avocado oil, apricot oil, meadowfoam seed oil, macadamia seed oil, oat kernel oil, palm seed oil, safflower oil, sandalwood oil, sesame oil, almond oil, wheat germ oil, cranberry oil and combinations thereof. Oils may be included in the composition in amounts of about 5% to about 65%.

Optionally, pharmaceutical actives such as anesthetics, antivirals, antibiotics, and analgesics, may be used in the lip treatment composition for example. The treatment strip is particularly applicable for cold sore treatments as the treatment can be dispensed without direct touch of the hand or fingers to the lips or affected area, and/or rubbing across the wound and smearing contamination to other portions of the lip and or the chance of cross contamination by re-using a stick and/or touching of the contents of a pot or tube with the hands.

Optionally, the lip treatment composition may comprise natural medicaments including, but not limited to, menthol, camphor, eucalyptus, salicylic acid, and derivatives of salicylic acid. Typically natural medicaments would be added in amounts of about .001% to about 10% and preferably less than about 3%. Amounts may vary depending on the potency of the medicament and the matrix in which the medicament is presented.

In some embodiments of lip treatment compositions it is desirable to avoid synthetic ingredients such as petroleum-derived materials and/or mammalian-derived materials and have a composition in which most of the materials (e.g. greater than 80%) are derived from botanical sources. The such botanical compositions may comprises moisturizing agents such as coconut oil, jojoba esters, and sunflower seed oil, waxes derived from plant sources and/or botanical butters, for example.

In general botanical butters contribute emollient and moisturizing skin-conditioning properties to the composition. In some embodiments, it is desirable to use a plurality of butters as these naturally derived substances have a variety of other attributes depending on the botanical source of the butter. For example, butters may vary in their sensory feel and/or have particular components with desired functionalities such as components that enhance lip barrier function, enhance penetration, or have antioxidant properties or the like.

Lip treatments compositions containing principally botanical derived materials, particularly botanical oils and butters are typically very soft. Thus, it is difficult to form a robust stick product having of high percentages of botanical oils and butters and pot or tube form tend to be very messy in application to the lips. The treatment strip is well suited to dispensing soft botanical based lip treatment compositions. As the treatment composition is applied to a substrate to form the treatment strips, significant amounts of oils and butters may be applied to the substrate. In use, the substrate both holds the treatment composition in place and provides a means for transferring the treatment composition to the lips with at least minimum contact with the user's hands and in some embodiments with grasping portions no contact with the user's hands.

Optionally, the lip treatment composition may comprise one or more of antioxidants, preservatives, flavorants, fragrances, colorants, cleansing agents, pH adjusting agents, and sensates. Antioxidants may protect the composition from oxidation (e.g. becoming rancid) and/or provide lip conditioning benefits upon application to the lips. Tocopherols, tocopheryl acetate, some botanical butters and green tea extracts are exemplary antioxidants suitable for use in the lip treatment composition. Exemplary preservatives include, but are not limited to, methylparaben, isopropylparaben, and isobutylparaben. Exemplary sensates include, but are not limited to, mint extracts, cinnamon extract, and capsaicin.

Other beneficial agents known to one skilled in the art may likewise optionally be included in the lip treatment compositions. Aloe extracts and natural organic acids are exemplary of other beneficial agents. Natural organic acids including α-hydroxy acids may act as exfoliants, for example. Lactic acid is an exemplary α-hydroxy acid.

### MANUFACTURE OF THE TOPICAL TREATMENT STRIP

The topical lip treatment strip of the invention is manufactured by applying a topical lip composition to a substrate. In an exemplary embodiment, the topical lip composition is prepared and the temperature and/or composition viscosity is adjusted such that the composition is in a flowable or, alternatively, a liquid form that can be dispensed onto the substrate and cover at least a portion of the substrate without excessive dripping or waste.

The viscosity of the topical lip composition may be reduced by raising the temperature to melt or partially melt additional components or, alternatively, solvents may be included in the composition to facilitate manufacture, for example. Alternatively, if the mixture is too thin to spread onto the substrate in the desired amount or drips, for example, viscosity modifying agents such as carboxymethylcellulose and or xanthan gum, for example, may be used to thicken the composition to a suitable viscosity. In a preferred embodiment, one or more grasping portions are provided at the side(s) of the treatment strip. In embodiments with grasping portions, placement of topical lip composition on the grasping portion is preferably avoided.

Any method of manufacture that permits controlled distribution of the topical lip composition on the one or more surfaces of the substrate may be suitable for manufacture of the tropical treatment strips. Such methods may for example include coating, spraying dipping, laminating or combinations thereof.

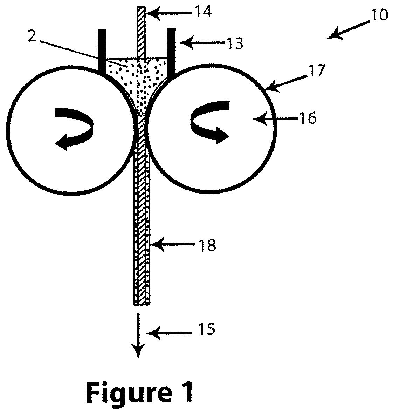

In one exemplary embodiment a coating process is used. The topical lip composition may be prepared by addition of all components at once or a sequential addition of components to a mixing vessel with stirring. Optionally, the mixture may be heated and/or viscosity adjusted to facilitate mixing and, for example. Referring to FIG. 1 upon completion of mixing the topical lip composition is transferred to a dispenser 13 where it is allowed to coat a substrate 14. The substrate 14 is moved in direction 15.

The substrate 14 in contact with topical lip composition 2 is rolled through the coater 17 in direction 15. In embodiment shown in FIG.1, the topical composition material 2 is deposited on both sides of the substrate 14. The thickness and width of composition 2 deposited on the substrate 14 is controlled by controlling parameters such as the width of the rollers 16 of the coater 17, and/or the pressure on the rollers 16 of the coater 17, and/or the viscosity of composition 2, and/or speed of rollers 16, for example. In one embodiment is it desirable to apply sufficient pressure to the rollers 16 of the coater 17 such that a portion of the topical lip composition 2 is embedded into the substrate 14. The coated substrate 18 exiting the coater 16 has topical lip composition on two surfaces of the coated substrate 18.

In one embodiment, the width of the substrate exceeds the width of the rollers of the coater 17, thus leaving a portion of the substrate uncoated and providing for grasping portion(s) on the edges of the coated strip. A single grasping portion may be used along one side or, alternatively, grasping portions may be left along multiple sides of the treatment strip. Once formed the coated substrate18 may be cut into individual single use treatment strip portions. Alternatively, the coated substrate may be formed into a roll(s) and cut or torn into single use treatment strips at the time of use.

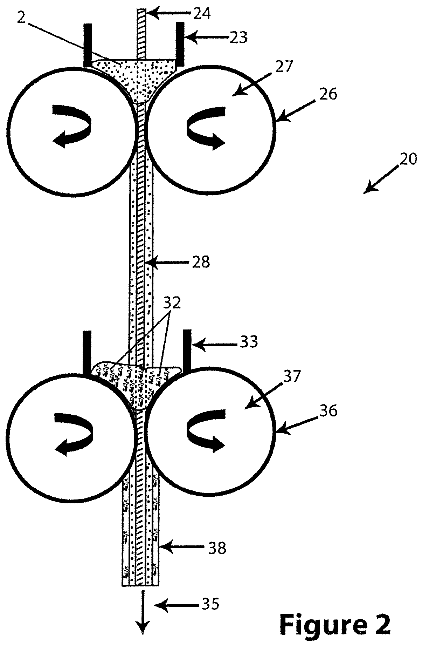

In one exemplary embodiment, a plurality of topical lip compositions may be applied to the substrate. Referring to FIG. 2, which is a diagram of a two layer coating system 20, a first topical composition 2 is transferred to the dispenser 23 where it is allowed to contact both sides of a substrate 24. The substrate 24 is moved in direction 35. The substrate 24 in contact with topical lip composition is rolled through the coater 26 in direction 35. The thickness and width of composition 2 deposited on substrate 24 is controlled by controlling parameters such as the width of the rollers 27 of the coater 26, and/or the pressure on the rollers 27 of the coater 26, and/or the viscosity of composition 2, and/or speed of rollers 27, for example. In one embodiment, it is desirable to apply sufficient pressure to the rollers of the coater 26 such that a portion of the topical composition 2 is embedded into the substrate 24. The coated substrate 28 exiting the coater 26 has topical composition 2 on two surfaces of the substrate 24. A second topical composition 32 is placed in dispenser 33 and the coated substrate 28 is advanced in direction 35. The coated substrate 28 in contact with topical lip composition 32 is rolled through the coater 36 in direction 35. Topical lip composition 32 is deposited on both sides of the coated substrate 28. The thickness and width of composition 32 deposited on coated substrate 28 is controlled by controlling parameters such as the width of the rollers of the coater 36, and/or the pressure on the rollers 37 of the coater 36, and/or the viscosity of composition 32, and/or speed of rollers 37, for example. The coated substrate 38 exiting the coater 36 has two layers of topical composition on each of two surfaces of the coated substrate 38. Once formed the coated substrate is cut into individual single use portions or alternatively formed into rolls.

In some embodiments the width of the substrate exceeds the width of the rollers 27, 37 of the coaters 26, 36. Thus leaving a portion of the substrate uncoated and providing for grasping portion(s) on the edges of the coated strip. A single grasping portion may be used along one side or alternatively grasping portions may be left along multiple side of the treatment strip.

As one skilled in the art will appreciate, additional layers of topical lip composition may be applied by increasing the number of dispenser and coater stations. Additionally, the compositions may the same or different. For example, multiple layers may be desirable when using actives and/or beneficial agents that lack or have limited compatibility. For example, different actives could be placed in different layers. Alternatively, multiple layer could be used with a barrier layer interposed between layers having incompatible components, for example. Alternatively, a multilayer system could include an upper most barrier layer to air and/or water access to inner layers with more reactive components, for example. Alternatively, a multilayer system could include three layers in which the first and third were the same and the middle layer different, for example.

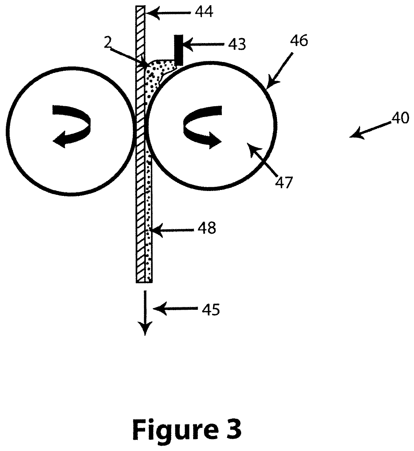

Referring to FIG. 3, in one embodiment, a topical lip composition 2 may be applied to one side of a substrate 44. The topical lip composition 2 is transferred to the dispenser 43 where it is allowed to contact one side of a substrate 44. The substrate 44 is moved in direction 45. The substrate 44 in contact with topical lip composition is rolled through the coater 46 in direction 45. The thickness and width of composition 2 deposited on substrate 44 is controlled by controlling parameters such as the width of the rollers 47 of the coater 46, and/or the pressure on the rollers 47 of the coater 46, and/or the viscosity of composition 2, and/or speed of rollers 47, for example. In one embodiment, it may be desirable to apply sufficient pressure to the rollers of the coater 46 such that a portion of the topical lip composition 2 is embedded into the substrate 44. The coated substrate 48 exiting the coater 46 has topical lip composition 2 on one surface of the coated substrate 44.

In some embodiments, the width of the substrate exceeds the width of the rollers 47 of the coater 46, thus leaving a portion of the substrate uncoated and providing for grasping portion(s) on the edges of the coated strip. A single grasping portion may be used along one side or, alternatively, grasping portions may be left along multiple sides of the treatment strip. Once formed the coated substrate is cut into individual single use treatment strip portions or alternatively formed into rolls.

As discussed above, one skilled in the art will appreciate multiple layers may be applied to the substrate by adding additional dispensers and roller stations. There is no requirement that both sides of the substrate have the same number of layers of topical lip composition, e.g., a coating process using a combination of coaters that variously includes coaters for coating a single side and coaters for coating both sides may be used. The multiple layer may be the same or different compositionally.

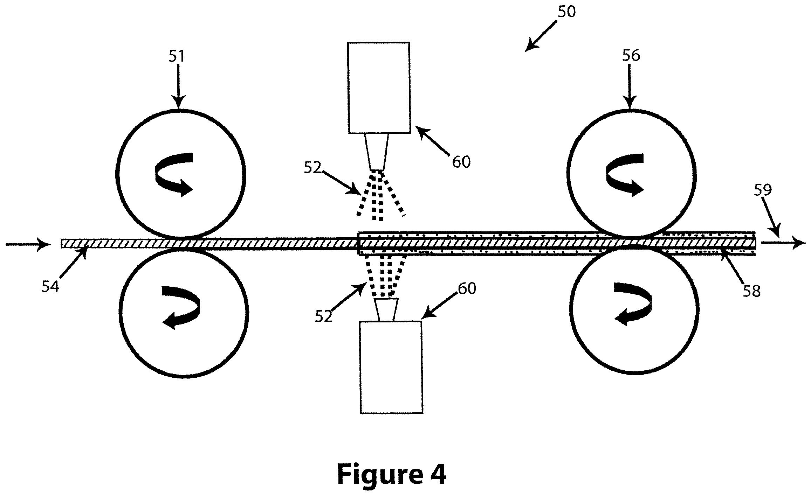

Referring to FIG. 4, in one embodiment, the topical lip composition 52 may be applied to the substrate 54 by spraying the topical lip composition 52 onto the substrate 54. The substrate is moved in direction 59 by rollers 51 and 56. A spraying station 50 is positioned between rollers 51 and 56. The spraying station 50 has reservoirs 60 equipped with spray nozzles which can dispense the topical lip composition 52 on to the substrate 54 in the form of a spray. The reservoirs 60 may contain the same or different compositions. Optionally different compositions may be applied to each side of the substrate simultaneously. Once the topical lip composition 52 is applied to the substrate the coated substrate 58 is advanced to rollers 56. The rollers 56 function to move the treatment strip and optionally may adjust the thickness and width of composition 52 deposited on substrate 54 which may be accomplished by controlling parameters such as the width of the rollers 56, and/or the pressure on the rollers 56, and/or the viscosity of composition 52, and/or speed of rollers 51, 56, for example. In one embodiment, it is desirable to apply sufficient pressure to the rollers of the coater 56 such that a portion of the topical composition 52 is embedded into the substrate 54. As shown in FIG. 4, the treatment strip 58 exiting roller 56 has topical composition 52 on two surfaces of the substrate 54. Once formed, the coated substrate is cut into individual single use treatment strip portions or alternatively formed into rolls.

One skilled in the art will appreciate that it is likewise possible to use a single reservoir with spray nozzle to deposit topical composition on only one side of a substrate. Alternatively, a plurality of spray stations may be used to apply multiple layers of topical composition to the substrate. The multiple layers may have the same or different components.

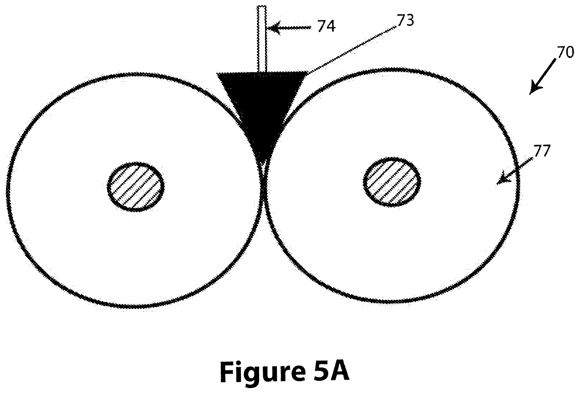

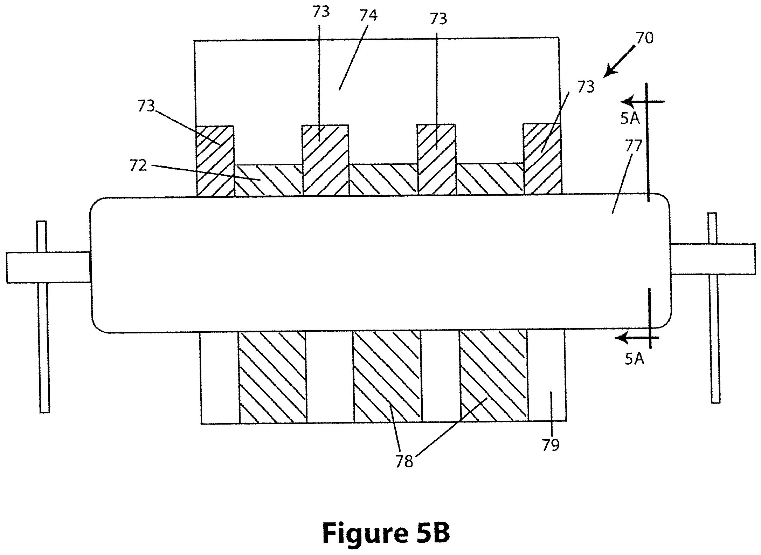

In one exemplary embodiment multiple strips may be formed simultaneously. An exemplary system for forming multiple strips simultaneously is shown in FIGs. 5A and 5B. FIG. 5A depicts the system in cross section and shows a substrate 74 being passed through a coater 70 having a plurality of reservoirs 73. Referring to the front view of the system shown in FIG. 5B, topical composition 72 is applied from each reservoir 73 to a portion of the substrate 74. The plurality of reservoirs 73 may contain the same or different topical lip compositions. The pair of rollers 77 function to move the substrate 74 and optionally may adjust the thickness and width of composition 72 deposited on substrate 74 which may be accomplished by controlling parameters such as the width of the rollers 77 of the coater, and/or the pressure on the rollers 77, and/or the viscosity of composition 72, and/or speed of rollers 77, for example. A portion of the substrate 74 may be coated 78 and portions of the substrate may remain uncoated 79. FIG. 5 shows a system which utilizes one coater and in which one layer of topical composition 72 is deposited on both sides or optionally one side of the substrate 74. As one skilled in the art will appreciate, additional layers of topical composition may be deposited by using a plurality of coaters assembled sequentially, for example.

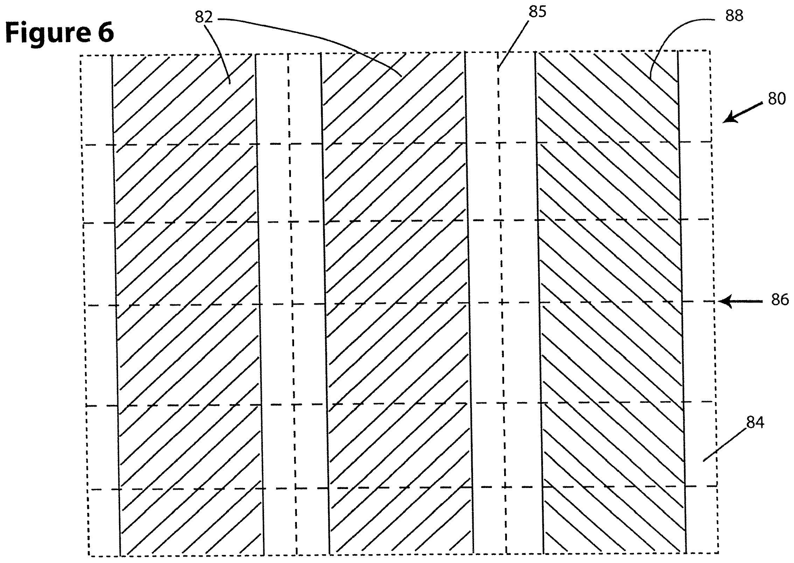

FIG. 6 shows an exemplary sheet of processed substrate 80 prepared in the coater 70 shown in FIG.5. The processed substrate 80 has coated regions 82 and uncoated regions 84. The sheet of processed substrate 80 may be cut into individual single use portions or individual treatment strips as shown by dotted lines 85, 86, for example. Alternatively, cuts may be made along only one of dotted lines 85 or 86 to form strips of a plurality of individual treatment strips that may be formed into rolls for packaging and distribution to users. Optionally, perforation may be formed along one or more of dotted lines 85 or 86 to facilitate dispensing individual treatment strips to a user.

The rectangular shape of the strip shown in FIG. 6 is exemplary. Other shapes or designs may be likewise suitable. In an exemplary embodiment of a lip treatment strip the coated portion 82, the length and width of the coated portion 82 of a single use portion is approximately the length and width of the human lips.

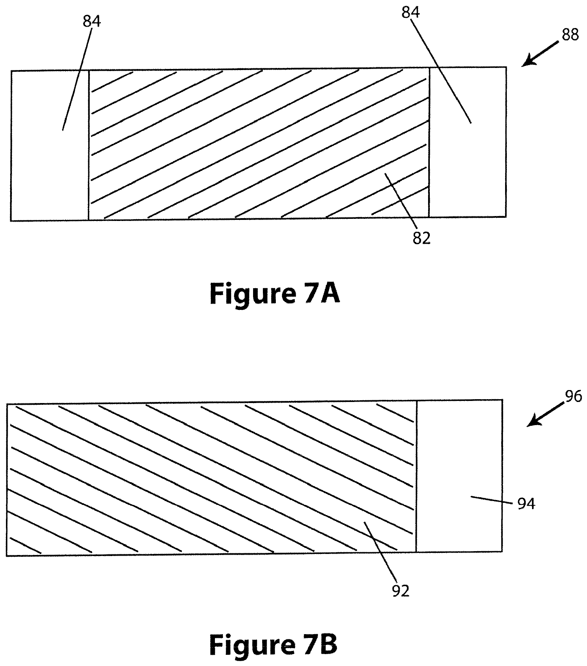

FIG. 7 shows two treatment strips for lip treatment 88, 96. The treatment strip in FIG. 7A has a coated 82 region and two uncoated regions 84. The two uncoated regions 84 are grasping regions for holding the treatment strip with the fingers when removing from a dispenser and/or positioning the treatment strips 88 between the lips for use. The embodiment shown in FIG. 7B shows a treatment strip 96 with a single coated portion 92 and a single uncoated region 94. The uncoated region 94 forms a grasping region for grasping the treatment strip with the fingers when removing from a dispenser and/or positioning the treatment strip 96 between the lips. Preferably, the grasping regions 84, 94 are of a sufficient size to permit grasping the treatment strip 88, 96 with minimal contact between the finger and the coating of the coated region 82, 92; and more preferably, the grasping regions 84, 94 are of a sufficient size to permit grasping the treatment strip 88, 96 with no contact between the fingers and the coating of the coated region 82, 92.

### EXAMPLES

The following examples further describe and demonstrate exemplary embodiments within the scope of the present invention. The examples are given solely for the purpose of illustration of the present invention. All exemplified amounts are weight/weight percentages unless otherwise specified.

### Example 1

A composition suitable for use for as the topical composition for lip topical treatment strips is provided in Table 1.

**TABLE 1**

| Ingredient | Amount % wt/wt |
|---|---|
| Yellow Beeswax | 3.50 |
| Shea butter | 0.75 |
| Coconut oil | 10.75 |
| Carnuauba wax | 1.25 |
| Candelilla wax | 13 |
| Flavorant | 4 |
| Mango butter | 0.75 |
| Tocopheryl acetate | 1 |
| Tocopherol | 0.2 |
| Avocado butter | 0.75 |
| Jojoba esters (mp 56-61° C) | 8 |
| Jojoba esters (mp 47-51° C) | 11 |
| Olive butter | 0.75 |
| Raspberry butter | 0.75 |
| Sunflower seed oil | 43.55 |

The composition of Table 1 may be prepared by combining the carnauba wax, candelilla wax, beeswax and coconut oil with mixing and heating in the range of 175-190 °F. Upon formation of a molten mixture the temperature may be reduced to 155-170°F and the jojoba esters added with mixing. Upon complete mixing the temperature may be reduced to 120-139°F and the sunflower oil added with mixing. The temperature may then be adjusted to 140-160 °F and the butters added with mixing. Upon completion of addition of the butters, tocopherol, tocopheryl acetate and flavorant may be added with mixing.

The composition of Table 1 may be placed in a dispenser and coated onto a substrate using roller coating equipment as described herein. A cellulosic material such as a paper or woven on non-woven material is exemplary of a material that may be used as a substrate. The composition of Table 1 should be in a liquid or semi-liquid state for coating.

## Claims

1. A topical lip treatment strip comprising:
a substrate comprising two surfaces; and
a topical lip treatment composition wherein the topical lip treatment composition is deposited onto the two surfaces of the substrate; and wherein each strip has a length of 1 cm to 10 cm and a width of 1 cm to 5 cm;
wherein one or more grasping portions are provided at the side(s) of the treatment strip on which no topical composition is deposited.

2. The topical lip treatment strip of Claim 1, wherein the topical lip treatment composition is at least partially absorbed on the substrate.

3. The topical lip treatment strip of Claim 1, wherein the substrate is selected from the group consisting of natural polymers, synthetic polymers, semi-synthetic polymers, fabrics, paper, woven material, non-woven material and combinations thereof.

4. The lip treatment strip of Claim 3, wherein the substrate is a biodegradable material.

5. The lip treatment strip of Claim 3, wherein the substrate hydrolyzes in the presence of moisture.

6. The topical lip treatment strip of Claim 1, wherein the topical lip treatment composition comprises at least one ingredient selected from the group consisting of beneficial agents, humectants, moisturizers, sunscreens and active agents and combinations thereof.

7. The topical lip treatment strip of Claim 1, wherein the topical lip treatment composition further comprises at least one active agent.

8. The topical lip treatment strip of Claim 1, comprising at least one second topical lip treatment composition wherein the second topical lip treatment composition is deposited over the topical lip treatment composition on at least one of the two surfaces.

9. A method of making a topical lip treatment strip as defined in claim 1, comprising:
preparing a topical lip treatment composition; and
depositing the topical lip treatment composition onto the two surfaces of a substrate comprising two surfaces, and avoiding deposition of the composition on the one or more grasping portions.

10. The method of Claim 9 further comprising pressing the topical lip treatment composition and the substrate together.

11. The method of Claim 9, further comprising depositing a layer of a second topical lip treatment composition onto the topical lip treatment strip.

12. The method of Claim 9, wherein the topical lip treatment composition is deposited onto the substrate using a method selected from the group consisting of coating, spraying, dipping, laminating and combinations thereof.

13. A kit for lip treatment comprising a resealable container having a plurality of topical lip treatment strips as defined in claim 1 deposited therein.

14. The kit of Claim 13 wherein the topical lip treatment strips are formed in a roll.

15. A method of using a topical lip treatment strip as defined in claim 1, comprising grasping the strip by the grasping portion, positioning the strip between the lips of a user, and pursing the lips against the strip so as to transfer the topical lip treatment composition to the lips of the user;
provided that the method is not a method for treatment of the human or animal body by therapy.

## Patentansprüche

1. Streifen zur topischen Lippenbehandlung, umfassend:
ein Substrat, das zwei Oberflächen umfasst, und
eine topische Lippenbehandlungszusammensetzung, wobei die topische Lippenbehandlungszusammensetzung an den zwei Oberflächen des Substrats abgeschieden ist, und wobei jeder Streifen eine Länge von 1 cm bis 10 cm und eine Breite von 1 cm bis 5 cm hat;
wobei ein oder mehrere Greifbereich(e) an der Seite / den Seiten des Behandlungsstreifens, auf dem / denen keine topische Zusammensetzung abgeschieden ist, bereitgestellt ist / sind.

2. Streifen zur topischen Lippenbehandlung gemäß Anspruch 1, wobei die topische Lippenbehandlungszusammensetzung wenigstens teilweise an dem Substrat absorbiert ist.

3. Streifen zu topischen Lippenbehandlung gemäß Anspruch 1, wobei das Substrat aus der Gruppe, bestehend aus natürlichen Polymeren, synthetischen Polymeren, halbsynthetischen Polymeren, Geweben, Papier, gewebtem Material, Vliesmaterial und Kombinationen davon, ausgewählt ist.

4. Streifen zur Lippenbehandlung gemäß Anspruch 3, wobei das Substrat ein bioabbaubares Material ist.

5. Streifen zur Lippenbehandlung gemäß Anspruch 3, wobei das Substrat in Gegenwart von Feuchtigkeit hydrolysiert.

6. Streifen zur topischen Lippenbehandlung gemäß Anspruch 1, wobei die topische Lippenbehandlungszusammensetzung wenigstens ein Ingrediens, ausgewählt aus der Gruppe, bestehend aus Mitteln mit vorteilhaften Eigenschaften, Feuchthaltemitteln, Befeuchtungsmitteln, Sonnenschutzmitteln und aktiven Agentien und Kombinationen davon, umfasst.

7. Streifen zur topischen Lippenbehandlung gemäß 1, wobei die topische Lippenbehandlungszusammensetzung außerdem wenigstens ein aktives Agens umfasst.

8. Streifen zur topischen Lippenbehandlung gemäß 1, umfassend wenigstens eine zweite topische Lippenbehandlungszusammensetzung, wobei die zweite topische Lippenbehandlungszusammensetzung über der topischen Lippenbehandlungszusammensetzung an wenigstens einer der zwei Oberflächen abgeschieden ist.

9. Verfahren zur Herstellung eines Streifens zur topischen Lippenbehandlung, wie in Anspruch 1 definiert, umfassend:
Herstellen einer topischen Lippenbehandlungszusammensetzung und
Abscheiden der topischen Lippenbehandlungszusammensetzung auf die zwei Oberflächen eines Substrats, das zwei Oberflächen umfasst, und Vermeiden einer Abscheidung der Zusammensetzung an dem einen oder den mehreren Greifbereichen.

10. Verfahren gemäß Anspruch 9, das außerdem Zusammenpressen der topischen Lippenbehandlungszusammensetzung und des Substrats umfasst.

11. Verfahren gemäß Anspruch 9, das außerdem Abscheiden einer Schicht einer zweiten topischen Lippenbehandlungszusammensetzung auf dem Streifen zur topischen Lippenbehandlung umfasst.

12. Verfahren gemäß Anspruch 9, wobei die topische Lippenbehandlungszusammensetzung unter Verwendung eines Verfahrens, ausgewählt aus der Gruppe, bestehend aus Beschichtung, Aufsprühen, Eintauchen, Laminieren und Kombinationen davon, auf dem Substrat abgeschieden wird.

13. Kit zur Lippenbehandlung, umfassend einen trennbaren Behälter, der darin eine Vielzahl von Streifen zur topischen Lippenbehandlung, wie in Anspruch 1 definiert, untergebracht hat.

14. Kit gemäß Anspruch 13, wobei die Streifen zur topischen Lippenbehandlung in einer Rolle geformt sind.

15. Verfahren zur Verwendung eines Streifens zur topischen Lippenbehandlung, wie in Anspruch 1 definiert, umfassend Greifen des Streifens an dem Greifbereich, Positionieren des Streifens zwischen den Lippen eines Verwenders und Schließen der Lippen gegen den Streifen, um so die topische Lippenbehandlungszusammensetzung auf die Lippen des Verwenders zu übertragen;
mit der Maßgabe, dass das Verfahren kein Verfahren zur Behandlung des Menschen- oder Tierkörpers durch Therapie ist.

## Revendications

1. Bande de traitement topique pour lèvres comprenant :
un substrat comprenant deux surfaces ; et
une composition de traitement topique pour les lèvres, dans laquelle la composition de traitement topique pour les lèvres est déposée sur les deux surfaces du substrat ; et dans laquelle chaque bande a une longueur de 1 cm à 10 cm et une largeur de 1 cm à 5 cm ;
dans laquelle une ou plusieurs parties de préhension sont prévues au niveau du ou des côtés de la bande de traitement sur lesquelles aucune composition topique n'est déposée.

2. Bande de traitement topique pour les lèvres selon la revendication 1, dans laquelle la composition de traitement topique pour les lèvres est au moins partiellement absorbée sur le substrat.

3. Bande de traitement topique pour les lèvres selon la revendication 1, dans laquelle le substrat est sélectionné à partir du groupe constitué par des polymères naturels, des polymères synthétiques, des polymères semi-synthétiques, des tissus, du papier, des matériaux tissés, des matériaux non tissés et des combinaisons de ceux-ci.

4. Bande de traitement pour les lèvres selon la revendication 3, dans laquelle le substrat est un matériau biodégradable.

5. Bande de traitement pour les lèvres selon la revendication 3, dans laquelle le substrat s'hydrolyse en présence d'humidité.

6. Bande de traitement topique pour les lèvres selon la revendication 1, dans laquelle la composition de traitement topique pour les lèvres comprend au moins un ingrédient sélectionné à partir du groupe par des agents bénéfiques, des humectants, des crèmes hydratantes, des crèmes solaires et des agents actifs et des combinaisons de ceux-ci.

7. Bande de traitement topique pour les lèvres selon la revendication 1, dans laquelle la composition de traitement topique pour les lèvres comprend en outre au moins un agent actif.

8. Bande de traitement topique pour les lèvres selon la revendication 1, comprenant au moins une seconde composition de traitement topique pour les lèvres, dans laquelle la seconde composition de traitement topique pour les lèvres est déposée pardessus la composition de traitement topique pour les lèvres sur au moins une des deux surfaces.

9. Procédé de fabrication d'une bande de traitement topique pour les lèvres selon la revendication 1, comprenant :
la préparation d'une composition de traitement topique pour les lèvres ; et
le dépôt de la composition de traitement topique pour les lèvres sur les deux surfaces d'un substrat comprenant deux surfaces, et l'évitement du dépôt de la composition sur les une ou plusieurs parties de préhension.

10. Procédé selon la revendication 9 comprenant en outre le fait de presser ensemble la composition de traitement topique pour les lèvres et le substrat.

11. Procédé selon la revendication 9 comprenant en outre le dépôt d'une couche d'une seconde composition de traitement topique pour les lèvres sur la bande de traitement topique pour les lèvres.

12. Procédé selon la revendication 9, dans lequel la composition de traitement topique pour les lèvres est déposée sur le substrat en utilisant un procédé sélectionné à partir du groupe constitué par le revêtement, la pulvérisation, l'immersion, la stratification et des combinaisons de ceux-ci.

13. Kit pour un traitement pour les lèvres comprenant un récipient amovible ayant une pluralité de bandes de traitement topique pour les lèvres selon la revendication 1 déposées dans celui-ci.

14. Kit selon la revendication 13, dans lequel les bandes de traitement topique pour les lèvres sont formées en rouleau.

15. Procédé d'utilisation d'une bande de traitement topique pour les lèvres selon la revendication 1, comprenant la préhension de la bande par la partie de préhension, le positionnement de la bande entre les lèvres d'un utilisateur, et pincer les lèvres contre la bande de façon à transférer la composition de traitement topique pour les lèvres aux lèvres de l'utilisateur ;
à condition que le procédé ne soit pas un procédé pour le traitement du corps humain ou animal par une thérapie.
